# EUROPÄISCHE PATENTSCHRIFT

(11) **EP 0 612 537 B1**
(45) Veröffentlichungstag und Bekanntmachung des Hinweises auf die Patenterteilung: **01.10.1997**
(21) Anmeldenummer: 93109618.4
(22) Anmeldetag: 16.06.1993
(51) Int. Cl.: A61M 39/00

(54) **Rückschlagventil, insbesondere für medizinische Anwendungen der Fluidtechnik**
Non-return valve, in particular for medical applications of fluid engineering
Valve anti-retour, en particulier pour utilisation médicale touchant à la technique des fluides

(30) Priorität: 18.02.1993 DE 4304949
(43) Veröffentlichungstag der Anmeldung: 31.08.1994
(73) Patentinhaber: Filtertek, S.A., F-60128 Plailly (FR)
(72) Erfinder: Myers, Jan Willem Marinus, NL-5913 Venlo (NL)
(74) Vertreter: Brose, D. Karl, Dipl.-Ing.

(56) Entgegenhaltungen:
- WO-A-91/11641
- DE-U- 9 209 491
- FR-A- 2 666 745
- US-A- 4 646 781

## Beschreibung

Es ist bekannt (DE 40 39 814 A1; US-A-4,996,199), ein Rückschlagventil aus einem zweiteiligen Gehäuse zu bilden, wobei ein erster Schlauchanschluß und ein zweiter Schlauchanschluß mit einer sich zwischen beiden Schlauchanschlüssen angeordneten Membranscheibe aus einem flexiblen Werkstoff vorgesehen sind. Bei Überdruck in dem ersten oder in dem zweiten Schlauchanschluß hebt die Membrane von einem Dichtsitz ab und es entsteht ein Durchflußquerschnitt. Bei entsprechendem Überdruck auf der dem Dichtsitz abgewandten Seite wird die Membranscheibe schnell und sicher auf den Dichtsitz gepreßt, so daß der freie Durchflußquerschnitt aufgehoben ist.

Die vorbekannte Lösung (DE 40 39 814 A1) strebt eine Verbesserung des Rückschlagventils in größerer Unabhängigkeit von Fertigungstoleranzen an, ein sicheres Öffnen und Schließen auch bei geringfügigen Druckdifferenzen. Hierzu bestehen die Lösungsmittel aus einem im Gehäuse eingespannten Randteil und aus einem zentralen Dichtteil der Membranscheibe, und eine diese beiden Teile miteinander verbindende dünnere Ringwand bildet die prinzipielle Basis. Sowohl der Dichtsitz und die Dichtwirkung als auch die Öffnungszeiten sind von der Einspannung der Membrane abhängig, die bei großen Fertigungstoleranzen unbestimmt wird. Eine Steigerung der Öffnungskraft durch radial äußere Beaufschlagung verbessert die Wirkungsfähigkeit der Membrane auch nicht. Bei der bekannten Anordnung kann die Vorspannung groß sein, was jedoch nur durch die Ausgestaltung mit einer dünneren Ringwand innerhalb der Membrane bewirkt werden kann. Der bekannte Vorschlag legt daher die Vorspannkraft in eine in der Dicke reduzierte Wandstelle der Membrane, so daß die absolute Vorspannung nicht sehr hoch sein kann.

Die andere bekannte Lösung (US-A-4,996,199) sieht eine nicht perforierte Membranscheibe vor, die lose ist, d.h. die Membranscheibe ist außen nicht etwa eingespannt und der Mittelteil und der Rand bewegen sich axial bei entsprechenden Betriebssituationen. Daher sind auch Elemente vorgesehen, die die Membranscheibe zentrisch halten. Eine entgegengesetzte Strömung lenkt den Rückfluß in die Mitte der stromabwärtsliegenden Oberfläche der Membranscheibe. Der Rückfluß hebt die Membranscheibe von Stützkanten, über die sie während des Vorwärtsflusses gebogen ist und verschließt sie gegenüber einer konkav abdichtenden Oberfläche. Diese Membranscheibe ist gegenüber hohem Druck in der Vorwärtsrichtung durch einen Flußumlenker und gegenüber starkem Rückflußdruck durch die konkave abdichtende Oberfläche geschützt. Bei Abwesenheit von vor- oder rückseitigem Fluiddruck nimmt die Membranscheibe eine neutrale Stellung ein, in der sie sich gegenüber dem Rückfluß durch Herstellung eines Linienkontaktes mit einer konvex gekrümmten Oberfläche, die sich auf der stromaufwärtigen Seite der Membranscheibe befindet, verschließt. Eine sichere und schnelle Abdichtung ist bei solchen Membranscheiben-Anordnungen nicht gewährleistet.

Derartige Rückschlagventile werden in der Medizin für Rohrleitungen von Fusions-Systemen, Diagnose-Ausrüstungen, Spritzen, intravenösen Schläuchen u.dgl. benötigt. Die an solche Rückschlagventile zu stellenden Anforderungen sind folgende:
a) diese Rückschlagventile müssen sehr sicher schließen und die Schließzeit darf nur Bruchteile von Sekunden betragen, um jeglichen Rückfluß von mit unerwünschten Stoffen belasteten Flüssigkeiten zu vermeiden und
b) die Herstellung muß nicht nur wirtschaftlich, sondern auch statistisch sehr genau sein.

Es sind strenge gesetzliche Vorschriften und Untersuchungen vorgesehen, um eine gleichbleibende und einheitliche Funktionssicherheit zu gewährleisten. Die Funktionssicherheit solcher Rückschlagventile wird z.B. durch den Technischen Überwachungsverein (TÜV) in Deutschland geprüft, und erst nach entsprechend positiven Ergebnissen erfolgt die Freigabe für die medizinische Anwendung.

In einem aus dem Dokument WO-A-91/11641 bekannten Rückschlagventil wird eine Membranscheibe durch Vorsprünge der jeweiligen Nabenteile zweier Schlauchanschlüsse festgehalten, die in entsprechend passende Einengungen in der ansonsten mit gleichmäßiger Dicke ausgebildeten Membranscheibe eingreifen. In diesem bekannten Dokument wird also nicht wie in der vorliegenden im Anspruch 1 definierten Erfindung vorgesehen, daß der äußere Umfangbereich der Scheibe durch einen von den Nabenteilen beider Schlauchanschlüsse gebildeten Hohlraum aufgenommen wird. Bei der bekannten Konstruktion aus WO-A-91/11641 haben ferner die ringförmigen Vorsprünge und die etwa gleichen ringförmigen schrägflächen in der Membranscheibe einen vorzugsweise dreieckigen Querschnitt.

Der im Anspruch 1 angegebenen Erfindung liegt das Problem zugrunde, ein schnell und sicher schließendes Rückschlagventil durch Aufbringen einer radialen Zugspannung in der Membranscheibe zu schaffen.

Die mit der Erfindung erzielten Vorteile bestehen insbesondere darin, daß bei günstiger Einstellung der Zugspannung ein noch schnelleres und gleich sicheres Ansprechen des Rückschlagventils stattfindet. Außerdem kann über die Einstellung der Zugspannung durch geänderte geometrische Gestaltung die Ansprechzeit und der Öffnungsdruck beeinflußt werden. Weiterhin ist die Herstellung der Einzelteile äußerst wirtschaftlich und statistisch sicher. Die Übertragung der höheren Vorspannung wird vorteilhafterweise bei ungeschwächter Membranscheibendicke in der Mitte und durch die leicht spritzbare Verdickung am Rand ermöglicht. Das eine ist für die Übertragung hoher Zugkräfte günstig und das andere für ein sicheres Einspannen .

Die Übertragung der Klemm- und Zugspannkräfte in radialer Richtung wird außerdem dadurch unterstützt, daß die Verdickung und die jeweiligen Wandflächen in den Nabenteilen mit etwa gleichen ringförmigen Schrägflächen versehen sind. Beim gegenseitigen Verschieben werden die Kräfte auf die Verdickung spielfrei übertragen. Aus herstelltechnischen sowie funktionstechnischen Gründen ist es vorgesehen, daß die Verdickung mit den Querschnitt begrenzenden Radien versehen ist.

Eine vorteilhafte Ausgestaltung der Erfindung sieht vor, daß die beiden Nabenteile mittels eines innen- und eines außenliegenden Ringvorsprungs ineinandergreifend verbindbar sind. Der Vorteil liegt in der Erzeugung einer Klemmkraft auf die Verdickung der Membranscheibe.

Weitere Vorteile ergeben sich ferner daraus, daß an dem ersten Nabenteil des ersten Schlauchanschlusses eine ringförmige Dichtlippe gebildet ist und daß an dem zweiten Nabenteil axial gegenüberliegend über den Umfang verteilt mehrere Hubbegrenzungsnoppen vorgesehen sind, wobei die Dichtlippe radial weiter innen zu Öffnungen liegt, die in der Membranscheibe angebracht sind.

Die Herstellung und die Funktionssicherung der zu montierenden Teile wird zusätzlich dadurch verbessert, daß gemäß der Erfindung jeweils die radial nach außen weisenden Wandflächen des Hohlraums einen im Querschnitt halbrund und ringförmigen Vorsprung an dem betreffenden Nabenteil bilden.

Die radiale Zugspannung wird ferner dadurch unterstützt, daß der ringförmige Vorsprung in radialer Ebene betrachtet gegenüber der Dichtlippe zurücksteht.

Ein Ausführungsbeispiel der Erfindung ist in der Zeichnung dargestellt und wird im folgenden näher beschrieben.

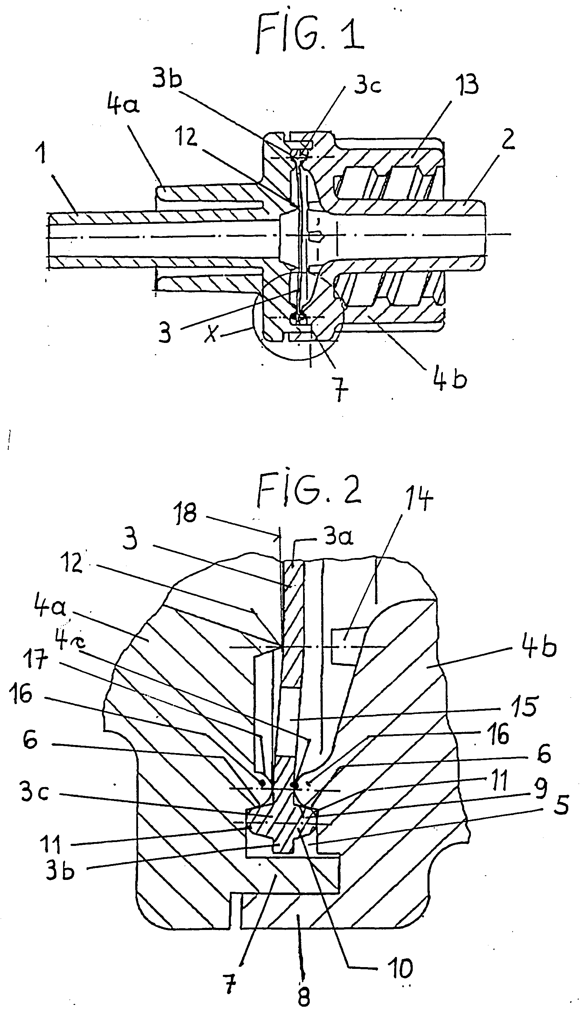

Es zeigen
- Fig.1: einen axialen Querschnitt durch das erfindungsgemäße Rückschlagventil im montierten Zustand und
- Fig.2: eine Einzelheit "X" aus Figur 1 in einem stark vergrößerten Maßstab.

Das Rückschlagventil ist insbesondere für medizinische Anwendungen der Fluidtechnik geschaffen. Es eignet sich insbesondere für Druckunterschiede von 0,1 bis o,oo2 bar (0.04 psig).
Es ist grundsätzlich aus einem ersten Schlauchanschluß 1 und aus einem zweiten Schlauchanschluß 2 zusammengesetzt. Zwischen den beiden Schlauchanschlüssen 1 und 2 ist eine Membranscheibe 3 aus einem flexiblen Werkstoff, wie z.B. Silikon, eingespannt. Die Membranscheibe 3 ist in ihrem Mittelteil 3a im wesentlichen gleichdick, so daß erhebliche Zugkräfte radial von innen nach außen und umgekehrt übertragen werden können. In ihrem äußeren Umfangsbereich 3b ist eine Verdickung 3c angespritzt, die ringförmig über den gesamten Umfang oder wenigstens in Umfangsabschnitten verläuft. Ein erstes Nabenteil 4a des ersten Schlauchanschlusses 1 und ein zweites Nabenteil 4b des zweiten Schlauchanschlusses 2 bilden einen Hohlraum 5 nach erfolgter Montage. Eine jeweils radial nach außen weisende Wandfläche 6 liegt derart gegen die Verdickung 3c an, daß bei Montage der beiden Nabenteile 4a und 4b in der Membranscheibe 3 radial gerichtete Zugkräfte übertragbar sind.

Hierbei sind die beiden Nabenteile 4a und 4b mittels eines innenliegenden Ringvorsprungs 7 an dem ersten Schlauchanschluß 1 und mittels eines außenliegenden Ringvorsprungs 8 an dem zweiten Schlauchanschluß 2 ineinandergreifend verbindbar. Die Verbindung kann z.B. durch Ultraschallschweißen erfolgen. Die Verdickung 3c und die jeweilige Wandfläche 6 (von dieser braucht nur eine vorhanden zu sein) in den Nabenteilen 4a und 4b sind mit etwa gleichen Schrägflächen 9 versehen. Die Verdickung 3c weist außerdem den Querschnitt 10 begrenzende Radien 11 auf.
In dem ersten Nabenteil 4a des ersten Schlauchanschlusses 1 ist eine ringförmige Dichtlippe 12 gebildet und an dem zweiten Nabenteil 4b, der als Luer-Lock-Anschluß 13 ausgebildet ist, sind, der Dichtlippe 12 axial gegenüberliegend, über den Umfang verteilt, mehrere Hubbegrenzungsnoppen 14 angeformt, wobei die Stirnfläche der Dichtlippe 12 radial weiter innen zu Öffnungen 15 liegen, die in der Membranscheibe 3 angebracht sind. Die jeweils radial nach außen weisenden Wandflächen 6 des Hohlraums 5 bilden einen im Querschnitt 16 halbrunden und gleichzeitig ringförmig verlaufenden Vorsprung 17 an dem betreffenden Nabenteil 4a oder 4b. Der ringförmige Vorsprung 17 steht in radialer Ebene 18 betrachtet gegenüber der Dichtlippe 12 zurück.

## Patentansprüche

1. Rückschlagventil, für medizinische Anwendungen, die Fluidiktechnik und für Druckunterschiede im Größenbereich von 0,1 bis 0,002 bar, mit einem ersten Schlauchanschluß (1) und einem zweiten Schlauchanschluß (2) und mit einer zwischen beiden Schlauchanschlüssen angeordneten Membranscheibe (3) aus flexiblem Werkstoff, welche bei Überdruck im Eingangsdurchlaß von einem Dichtsitz (12) abhebbar ist, und welche bei Überdruck im Ausgangsdurchlaß sicher und in Minimalzeiten auf den Dichtsitz (12) andrückbar ist, wobei der erste Schlauchanschluß (1) an einem ersten Nabenteil (4a) und der zweite Schlauchanschluß (2) an einem zweiten Nabenteil (4b) ausgebildet ist, und wobei die Nabenteile (4a, 4b) stirnseitig miteinander verbunden ein Ventilgehäuse bilden und die Membranscheibe (3) zwischen den Nabenteilen (4a, 4b) gehalten ist, wobei die Membranscheibe (3) im Mittelteil im wesentlichen gleichmäßig dick ausgebildet und an ihrem äußeren Umfangsbereich (3b) mit einer ringförmigen Verdickung (3c) versehen ist, wobei das erste Nabenteil (4a) des ersten Schlauchanschlusses (1) und das zweite Nabenteil (4b) des zweiten Schlauchanschlusses (2) einen die Verdickung (3c) allseitig mit Ausnahme eines Durchlasses (4c) in Größe der Dicke des Mittelteils (3a) aufnehmenden Hohlraum (5) bilden, daß eine radial innenliegende, nach außen weisende Wandfläche (6) des Hohlraums (5) derart gegen die Verdickung (3c) anliegt, daß beim Zusammenfügen der beiden Nabenteile (4a, 4b) zu dem Ventilgehäuse in der Membranscheibe (3) radial gerichtete Zugkräfte erzeugbar sind, wobei die Verdickung (3c) und die nach außen weisenden Wandflächen (6) in den Nabenteilen (4a, 4b) mit etwa gleichen ringförmigen Schrägflächen (9) versehen sind, wobei die Verdickung (3c) mit ihrem Querschnitt (10) begrenzenden Radien (11) versehen ist, und wobei die radial nach außen weisenden Wandflächen (6) des Hohlraums (5) einen im Querschnitt (16) halbrunden und ringförmigen Vorsprung (17) an dem dazugehörigen Nabenteil (4a oder 4b) bilden.

2. Rückschlagventil nach Anspruch 1, dadurch gekennzeichnet, daß an dem zweiten Nabenteil (4b) eines Luer-Lock-Anschlusses (13) über den Umfang verteilt mehrere Hubbegrenzungsnoppen (14) vorgesehen sind, welche einer an dem ersten Nabenteil (4a) des ersten Schlauchanschlusses (1) ausgebildeten ringförmigen Dichtlippe (12) axial gegenüberliegend angeordnet sind.

3. Rückschlagventil nach Anspruch 1 oder 2, dadurch gekennzeichnet, daß die Dichtlippe (12) gegenüber dem ringförmigen Vorsprung (17) in radialer Ebene (18) vorsteht.

4. Rückschlagventil nach einem der vorstehenden Ansprüche, dadurch gekennzeichnet, daß die beiden Nabenteile (4a, 4b) mittels eines innenliegenden Ringvorsprungs (7) und eines außenliegenden Ringvorsprungs (8) verbunden sind, wobei die Ringvorsprünge (7 und 8) ineinander eingreifen.

## Claims

1. A non-return valve for medical applications, fluid technology and for pressure differences of the order of magnitude from 0.1 to 0.002 bar, with a first hose connection (1) and a second hose connection (2) and with a diaphragm disc (3) made of flexible material arranged between both hose connections and adapted, in the event of excess pressure in the intake hose connection, to lift off a sealing seat (12) and, in the event of excess pressure in the outlet hose connection, to be applied securely and in minimum time to said sealing seat (12), the first hose connection (1) being arranged on a first hub part (4a) and the second hose connection (2) on a second hub part (4b) and the two hub parts (4a,4b) when mutually engaged at their frontal sides forming a valve body and the diaphragm disc (3) being retained between the hub parts (4a,4b), with the diaphragm disc (3) being of substantially even thickness in its middle portion and provided in its outer circumferential region (3b) with an annular bulge (3c) and the first hub part (4a) of the first hose connection (1) and the second hub part (4b) of the second hose connection (2) together forming a cavity (5) receiving the bulge (3c) all round with the exception of a passage gap (4c) of a size corresponding to the thickness of the middle portion (3a),with a radially inwardly situated outwardly directed wall face (6) of the cavity (5) engaging with the bulge (3c) in such a manner that during the joining together of the two hub parts (4a,4b) to make up the valve body radially directed tension forces can be generated in the diaphragm disc (3), the bulge (3c) and the outwardly directed wall faces (6) in the hub parts (4a,4b) being provided with approximately equal annular inclined faces (9) and the bulge (3c) being provided with radii(11) limiting its cross-section (10), and wherein the radially outwardly directed wall faces (6) of the cavity (5) form an annular projection (17) of semicircular cross section (16) on the associated hub part (4a or 4b).

2. A non-return valve according to claim 1,characterised in that on the second hub part (4b) of a Luer-Lock connector (13) there are provided several circumferentially spaced lift-limiting knubs (14) which are situated axially opposite an annular sealing lip (12) formed on the first hub part (4a) of the first hose connection (1).

3. A non-return valve according to claim 1 or 2,characterised in that the sealing lip (12) projects in the radial plane (18) relative to the annular projection (17).

4. A non-return valve according to any one of the preceding claims, characterised in that the two hub parts (4a,4b) are mutually connected by means of an internally situated annular projection (7) and an externally situated annular projection (8), the annular projections (7 and 8) interengaging one inside the other.

## Revendications

1. Clapet anti-retour, pour des applications médicales, la technique hydraulique et pour des différences de pression d'un ordre de grandeur compris entre 0,1 et 0,002 bar, avec un premier raccordement pour tuyaux (1) et un deuxième raccordement pour tuyaux (2) et avec un disque membrane (3) en matériau flexible, placé entre les deux raccordements pour tuyaux, qui, en cas de surpression dans le passage d'entrée, peut être soulevé d'un siège d'étanchéité (12), et qui, en cas de surpression dans le passage de sortie, peut être pressé sur le siège d'étanchéité (12), de façon sûre et en des temps minimaux, le premier raccordement pour tuyaux (1) étant formé sur une première partie de moyeu (4a) et le deuxième raccordement pour tuyaux (2), sur une deuxième partie de moyeu (4b), et les parties de moyeu (4a, 4b), reliées entre elles frontalement, formant un boîtier de soupape et le disque membrane (3) étant maintenu entre les parties de moyeu (4a, 4b), le disque membrane (3) présentant dans la partie médiane une épaisseur sensiblement régulière et étant pourvu d'un épaississement annulaire (3c), dans sa zone périphérique extérieure, la première partie de moyeu (4a) du premier raccordement pour tuyaux (1) et la deuxième partie de moyeu (4b) du deuxième raccordement pour tuyaux (2) formant un espace creux (5) recevant l'épaississement (3c) de tous côtés, à l'exception d'un passage (4c) de dimensions correspondant à l'épaisseur de la partie médiane (3a), une surface de paroi (6) radialement intérieure, tournée vers l'extérieur, de l'espace creux (5), s'appliquant contre l'épaississement (3c) de manière que lors de l'assemblage des deux parties de moyeu (4a, 4b) pour former le boîtier de soupape, des forces de traction dirigées radialement peuvent être produites dans les disque membrane (3), l'épaississement (3c) et les surfaces de paroi (6), dirigées vers l'extérieur, étant pourvus de surfaces obliques (9) annulaires à peu près identiques, l'épaississement (3c) étant pourvu de rayons (11) délimitant sa section transversale (10), et les surfaces de paroi (6), dirigées radialement vers l'extérieur, de l'espace creux (5), formant une saillie (17) de section transversale (16) en demi-cercle et annulaire, sur la partie de moyeu (4a ou 4b) respective.

2. Clapet anti-retour selon la revendication 1, caractérisé en ce que sur la deuxième partie de moyeu (4b) d'un raccordement à verrouillage de Luer (13) sont prévus plusieurs boutons de limitation de course (14), répartis sur la périphérie, qui font face axialement à une lèvre d'étanchéité (12) de forme annulaire, formée sur la première partie de moyeu (4a) du premier raccordement pour tuyaux (1).

3. Clapet anti-retour selon la revendication 1 ou 2, caractérisé en ce que la lèvre d'étanchéité (12) dépasse, dans le plan radial (18), de la saillie (17) annulaire.

4. Clapet anti-retour selon l'une des revendications précédentes, caractérisé en ce que les deux parties de moyeu (4a, 4b) sont assemblées au moyen d'une saillie annulaire intérieure (7) et d'une saillie annulaire extérieure (8), les saillies annulaires (7 et 8) s'engageant l'une dans l'autre.
